Europäisches Patentamt

European Patent Office

Office européen des brevets

(11) Numéro de publication: **0 396 458**
**A1**

(12) **DEMANDE DE BREVET EUROPEEN**

(21) Numéro de dépôt: **90401161.6**

(22) Date de dépôt: **27.04.90**

(51) Int. Cl.⁵: **C07C 313/04, C07C 303/32,**
**C07C 309/06**

(30) Priorité: **02.05.89 FR 8905809**

(43) Date de publication de la demande:
**07.11.90 Bulletin 90/45**

(84) Etats contractants désignés:
**AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Forat, Gérard**
**276, rue du Guesclin**
**F-69003 Lyon(FR)**
Inventeur: **Langlois, Bernard**
**91, rue du Guesclin**
**F-69006 Lyon(FR)**
Inventeur: **Lorieu, Philippe**
**Le Balzac, 6 rue des Frères**
**F-69100 Villeurbanne(FR)**
Inventeur: **Poppei, Jean**
**Le Clos**
**F-38290 Frontonas(FR)**

(74) Mandataire: **Ricalens, François et al**
**RHONE-POULENC CHIMIE Service Brevets**
**Chimie 25, quai Paul Doumer**
**F-92408 Courbevoie(FR)**

(54) **Procédé de purification des trifluoromethanesulfinate et sulfonate de sodium.**

(57) La présente invention concerne un procédé de purification du trifluorométhanesulfinate de sodium.
Ce procédé est caractérisé par le fait qu'il comporte l'étape de mise en contact d'un mélange de solides contenant au moins un sel de l'acide halohydrique et un sel de l'acide triflinique avec une phase organique contenant :

- a) un solvant polaire non miscible à l'eau en toute proportion choisi parmi le groupe constitué par les alcools, les cétones et les esters dont le point d'ébullition sous pression atmosphérique est inférieur à 200° C, de préférence à 150° C, et leurs mélanges ;
- b) un solvant aromatique choisi dans le groupe des solvants dont le point d'ébullition est inférieur à 200° C, de préférence à 150° C et leurs mélanges.

Application à la synthèse de l'acide triflique.

## PROCEDE DE PURIFICATION DES TRIFLUOROMETHANESULFINATE ET SULFONATE DE SODIUM

La présente invention concerne un procédé de purification du trifluorométhanesulfinate de sodium, encore appelé triflinate de sodium, et du trifluorométhanesulfonate de sodium, encore appelé triflate de sodium, ainsi que de l'acide trifluorométhanesulfonique, encore appelé acide triflique.

Il est connu de préparer selon le brevet EP 237 446 les acides perhalogénométhanesulfiniques et sulfoniques ainsi que leurs sels par mise en contact dans un solvant polaire, de préférence dans le diméthylformamide, d'un dithionite alcalin ou alcalino-terreux ou d'un hydroxyméthanesulfinate avec le bromure de trifluorométhyle.

Le trifluorométhanesulfinate est isolé, après évaporation des solvants, par extraction à l'acétate d'éthyle. Par cette méthode, il subsiste toujours une quantité d'eau et de diméthylformamide non négligeable dans le triflinate ce qui pose des problèmes pour son conditionnement ou pour les étapes ultérieures d'accès à l'acide triflique.

Le trifluorométhanesulfonate est préparé à partir du trifluorométhanesulfinate par oxydation à l'eau oxygénée suivie d'une évaporation de la solution aqueuse et d'une extraction des cristaux à l'acétone.

Si ces procédés de purification sont faciles à mettre en oeuvre au niveau d'un essai de laboratoire, ils sont en revanche difficiles à transposer au niveau industriel pour des problèmes de sécurité et de rentabilité.

La présente invention a pour objet un procédé industriel d'extraction et de purification du trifluorométhanesulfinate à partir du bromure de trifluorométhyle par exemple selon le procédé décrit dans le brevet EP 237 446.

En particulier, les aspects technico-économiques, tels que par exemple la consommation de réactifs ou le rendement joue un rôle plus important. D'autre part, il est parfois intéressant d'enchaîner les réactions sans isoler le produit à chaque étape.

Selon ce procédé, à la fin de la réaction de synthèse, on se trouve en présence d'un mélange réactionnel contenant différents sels dont notammment:
- le bromure de sodium (6),
- le phosphate monosodique (8),
- le phosphate disodique (7),
- le sulfite de sodium $Na_2SO_3$,
- l'hydrogénosulfite de sodium $NaHSO_3$
- le thiosulfate de sodium (4),
- le trifluorométhanesulfinate de sodium (5),
dans un solvant utilisé comme milieu réactionnel en particulier le mélange d'eau et de diméthylformamide.

La concentration du trifluorométhanesulfinate de sodium (5) est souvent inférieure à 10 % en poids, les autres sels représentant environ 20 % en poids du milieu réactionnel.

L'extraction d'une faible quantité en poids (10 %) de sels mélangés à de nombreux autres sels a toujours été un gros problème dans l'industrie. Dans le cas particulier des procédés de trifluorométhylsulfinate préparés à partir de bromure de trifluorométhyle, la séparation de ces derniers d'avec les iodures présentent un problème délicat d'autant que les ions iodures sont très gênants lors de l'oxydation des trifluorométhylsulfinates, en trifluorométhylsulfinate ou triflate. En particulier le rapport massique entre le brome et l'ion trifluorométhylsulfinate doit être au plus égal à 5 %, avantageusement à 2%, de préférence à 1 %.

Les autres sels quoique moins gênants, constituent des impuretés qu'il est préférable d'éliminer dans la mesure du possible.

Enfin, il convient de mentionner que l'acide triflinique et les triflinates sont relativement peu stables et qu'il convient d'éviter de les soumettre à des traitements trop brutaux et qu'en particulier il convient d'éviter de trop chauffer le milieu réactionnel. En outre, notamment pour récupérer les solvants non aqueux, on chasse les solvants constitutifs du milieu réactionnel ou de la part du milieu réactionnel contenant le sel de l'acide triflinique, en général le sel de sodium. Ainsi, il convient de traiter un mélange de sels sous forme solide pour en extraire aussi sélectivement que possible le sel d'acide triflinique.

C'est pourquoi un des buts de la présente invention est de fournir un procédé de récupération de l'acide triflinique ou de ses sels qui permettent de séparer l'ion triflinate d'avec l'ion bromure.

Un autre but de la présente invention est un procédé du type précédent qui permette la séparation de l'ion triflinate d'avec les autres sels minéraux.

Un autre but de la présente invention est de fournir un procédé du type précédent qui permet de traiter les matières solides.

Un but complémentaire de la présente invention est de fournir un procédé d'obtention desdites matières

EP 0 396 458 A1

solides qui facilitent la récupération ultérieure de l'ion triflinate.

Ce but et d'autres qui apparaîtront par la suite sont atteints au moyen d'un procédé de récupération de l'acide triflinique caractérisé par le fait qu'il comporte l'étape de mise en contact d'un mélange de solides contenant au moins un sel de l'acide halohydrique et un sel de l'acide triflinique avec une phase organique contenant :

- a) un solvant polaire non miscible à l'eau en toute proportion choisi parmi le groupe constitué par les alcools, les cétones et les esters dont le point d'ébullition sous pression atmosphérique est inférieur à 200° C, de préférence à 150° C, et leurs mélanges ;

- b) un solvant aromatique choisi dans le groupe des solvants dont le point d'ébullition est inférieur à 200° C, de préférence à 150° C et leurs mélanges. La phase organique doit être liquide à température ambiante et de préférence à 0° C.

Le rapport entre les solvants A et les solvants B est de préférence compris entre 1/5 et 5 en masse de préférence entre 2 et 1/5. Dans le cas du couple acétate d'éthyle toluène, la zone optimale du rapport acétate d'éthyle sur toluène varie entre 2 et 1.

Les éléments autres que le solvant A et le solvant B contenus dans la phase organique représentent au plus 1/4 avantageusement 1/10 de préférence 1/5 de la masse totale de la phase organique.

Il est à noter que la présence de solvants B facilite considérablement les étapes de filtration qui permettent de séparer la phase organique chargée en ions triflinates des sels restés insolubles.

En outre, selon la présente invention, il a été montré que suivant la manière dont les solvants du milieu réactionnel avaient été chassés, les solides essentiellement formés de sels contenant l'acide triflinique était plus ou moins facilement extractible par ladite phase organique.

Ainsi lorsque le dernier solvant à être évaporé est un solvant organique, en particulier du diméthylformamide (DMF), ou bien la filtration subséquente à l'extraction solide-liquide ou bien l'extraction elle-même ou bien les deux se réalisent dans de mauvaises conditions et donnent de médiocres résultats et rendement.

En revanche lorsque le dernier liquide à être évaporé est de l'eau, l'extraction solide-liquide se passe dans d'excellentes conditions avec de bons rendements.

En outre, dans ce dernier cas la quantité de solvant organique résiduel dans le gâteau est moins importante et rique moins de perturber les étapes ultérieures de purification de l'acide triflinique et de son éventuelle oxydation en acide trifluorométhylsulfonique, ou triflique. Les différentes mises en oeuvre de la présente invention seront décrites en se référant au rhéogramme de la figure I.

Ces mises en oeuvres prennent comme exemple de solvants non aqueux dans le mélange réactionnel le diméthylformamide.

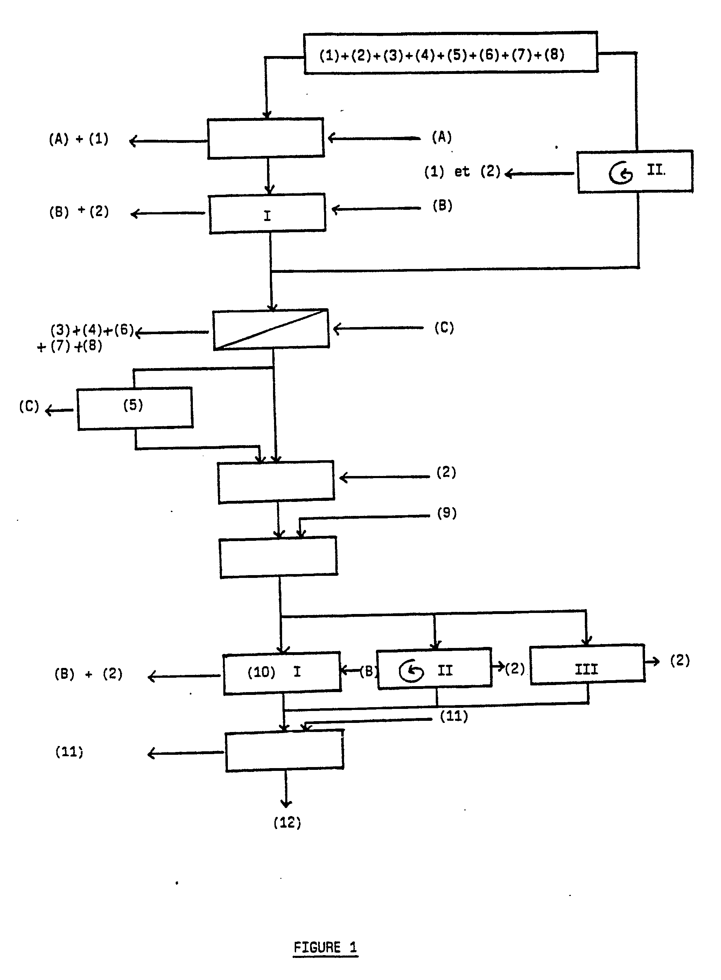

Selon une première façon de mettre en oeuvre, conformément à la figure 1, le procédé d'extraction du trifluorométhanesulfinate de sodium, on extrait d'abord le diméthylformamide (1) du mélange réactionnel par le dichlorométhane (A) selon un procédé d'extraction liquide-liquide, puis la solution résultante est déshydratée par entraînement azéotropique à l'aide de toluène (B). Ce toluène pourra servir d'éléments constitutifs de la phase organique d'extraction solide-liquide. On obtient alors une suspension pâteuse ou un solide presque entièrement déshydraté de trifluorométhanesulfinate de sodium.

La première façon de mettre en oeuvre le procédé d'extraction est réalisée en continu.

Il a été remarqué que l'extraction du solvant polaire et notamment du DMF par le chlorure de méthylène était souvent extrêmement difficile et impraticable au plan industriel. Ces difficultés sont levés par une étape préalable d'ajustement de la teneur en eau de la phase chargée en triflinate à extraire.

Dans cette étape on ajuste la teneur en eau de manière que la masse de l'eau dans le mélange à extraire soit au moins égale à 2 fois avantageusement 2,5 fois la masse des sels dissous y compris le triflinate ; de préférence entre 2,5 et 3.

Selon une deuxième façon de mettre en oeuvre, conformément à la figure 1, le procédé d'extraction du trifluorométhylsulfinate de sodium, on évapore conjointement le diméthylformamide (1) et l'eau (2) dans une turbosphère.

L'évaporation se fait à une température inférieure à 100° C, de préférence comprise entre 50 et 70° C au sein du liquide, et sous une pression réduite à environ 400 à 800 pascals. Le solide obtenu, est entièrement déshydraté et débarassé du diméthylformamide.

Le solide obtenu par la première ou la seconde méthode de mise en oeuvre est ensuite mis en solution dans ladite phase organique qui sera par la suite désignée sous la forme simplifiée d'acétate d'éthyle (C).

On introduit une quantité de phase organique constituée d'acétate d'éthyle et de toluène en quantité telle que la masse d'acétate d'éthyle corresponde à environ 2 à 10 fois le poids de solide obtenu à l'étape précédente.

La solubilisation du trifluorométhanesulfinate de sodium est effectuée avantageusement à relativement basse température, en général à la température ambiante (20° plus ou moins 5° C), ou à une température

3

comprise entre l'ambiante et 50° C.

La suspension obtenue est filtrée permettant d'éliminer l'ensemble des sels qui sont insolubles dans l'acétate d'éthyle tels que :
- le bromure de sodium (6),
- le phosphate disodique (8),
- le phosphate monosodique (7),
- le sulfite de sodium (3),
- l'hydrogénosulfite de sodium (3),
- le thiosulfate de sodium (4).

Le trifluorométhanesulfinate de sodium (5) en solution dans l'acétate d'éthyle est récupérée par distillation de l'acétate d'éthyle au moyen d'une colonne ou d'une turbosphère.

On préfère utiliser une turbosphère dont les parois sont chauffées à environ 50° C et sous une pression d'environ 400 à 800 pascal.

Le sulfinate sec (5) obtenu présente un taux d'humidité inférieur à 10% en poids.

Le trifluorométhanesulfinate sec ou sa solution dans l'acétate d'éthyle peut être utilisé par la synthèse du trifluorométhanesulfonate (10).

La poudre de sulfinate ou la solution dans l'acétate d'éthyle est mise en contact avec une quantité d'eau (2) correspondant à environ deux fois le poids du sulfinate.

On obtient alors une solution aqueuse à environ 50 % en poids de sulfinate.

Cette solution est mise en contact avec une quantité de péroxyde d'hydrogène (9) correspondant à environ 10 % d'excès par rapport à la stoechiométrie permettant d'oxyder le trifluorométhanesulfinate en trifluorométhanesulfonate selon la réaction :

$$CF_3 SO_2 Na + H_2 O_2 \text{ --------------- } CF_3 SO_3 Na + H_2O$$

Cette oxydation peut aussi être réalisée par l'hypochlorite de sodium selon la réaction :

$$CF_3 SO_2 Na + NaOCl \text{ --------------- } CF_3 SO_3 Na + NaCl$$

Le trifluorométhanesulfonate (10) est séché, à partir de sa solution aqueuse, par au moins trois méthodes :
- soit par distillation azéotropique de l'eau avec le toluène [I],
- soit par séchage à l'aide d'une turbosphère [II],
- soit par séchage par atomisation [III].

Dans le cas où l'oxydation est réalisée au moyen de l'hypochlorite de sodium on pourra éliminer après le séchage par un des moyens (I), (II) ou (III) le chlorure de sodium formé. Cette élimination est effectuée par extraction du sulfonate à l'acétate d'éthyle et évaporation de l'acétate d'éthyle.

Dans le cas où l'oxydation est réalisée avec le péroxyde d'hydrogène préalablement à l'étape de séchage, il est souvent nécessaire d'éliminer l'eau oxygénée subsistante pour éviter tout risque éventuel d'explosion.

Cette élimination est effectueée par distillation préalable de l'eau oxygénée et de l'eau en l'absence de toluène puis, ajout du toluène. Cette élimination peut également être réalisée par addition d'un réducteur. On peut citer parmi les réducteurs utilisables le sulfate ferreux, le dithionite de sodium, le bisulfite de sodium.

Selon un exemple de mise en oeuvre, on engage environ 0,2 moles de réducteur par kilo de masse à traiter.

L'acide trifluorométhanesulfonique pur (12) est obtenu à partir du trifluorométhanesulfonate de sodium par acidification avec un oléum sulfurique (11) contenant environ 50 % d'excès molaire de trioxyde de soufre par rapport à l'eau présente dans le trifluorométhanesulfonate de sodium. On utilise environ 2 moles d'acide sulfurique par mole de trifluorométhanesulfonate de sodium introduite. L'acide trifluorométhanesulfonique anhydre est ensuite obtenu par distillation à une température de 51° C sous 130 Pascals.

Lorsque la distillation est effectuée à partir d'une solution d'acide sulfurique exempte d'oléum, on obtient uniquement le monohydrate de l'acide trifluorométhanesulfonique qui présente une acidité bien inférieure à celle de l'acide trifluorométhanesulfonique anhydre.

La présente invention sera plus complètement décrite à l'aide des exemples suivants qui ne doivent pas être considérés comme limitatifs de l'invention.

EXEMPLE 1

1-1 Synthèse du trifluorométhanesulfinate de sodium (ou triflinate de sodium)

Dans un réacteur de 40 litres préalablement balayé à l'azote, étanche sous 15 bars, on charge sous agitation (100 tours/minute) :
- 15 Kg d'eau permutée,
- 3,3 Kg de phosphate disodique.

On chauffe le milieu à 50° C pour dissoudre le phosphate.

On charge ensuite 4,8 Kg de dithionite de sodium brut et 9,5 Kg de diméthylformamide (DMF).

On purge deux fois le réacteur sous 10 bars de CF₃Br, on l'isole et on porte l'agitation à 200 tours/minute.

On introduit alors CF₃Br sous 13 bars tout en chauffant le milieu réactionnel à 65° C. La pression de Cf₃Br est maintenue entre 13 et 15 bars jusqu'à la fin de l'absorption.

Ensuite, on refroidit la masse réactionnelle à 30° C et on laisse décanter une heure.

La phase inférieure (4,8 kg) contenant les phosphates est régénérée par de la soude puis recyclée après addition d'un complément éventuel pour atteindre le taux de phosphate désiré.

La phase supérieure (30,3 kg) contient le trifluorométhanesulfinate de sodium et divers composés dont :

| - DMF = | 9,5 kg, | |
|---|---|---|
| - eau = | 12,7 kg, | |
| - impuretés salines = | 5 kg, | |
| - triflinate de sodium = | 3,1 kg | (rendement par rapport au dithionite introduit = 83,4 %) |

1-2 Traitement du brut réactionnel selon le premier mode de mise en oeuvre

* Elimination du DMF

Dans un réacteur de 100 litres respirant sous azote, on charge :
- le brut réactionnel précédent (30,3 kg),
- 5 kg d'eau permutée,
- 30,3 kg de dichlorométhane.

On agite à 200 tours/minute pendant 10 minutes, on laisse décanter 20 minutes et on élimine la phase inférieure qui pourra être recyclée.

On recharge dans le réacteur contenant la phase supérieure précédente 20 kg de CH₂Cl et on répète l'extraction précédente. On renouvelle cette extraction 8 fois. Le rendement d'extraction du DMF après la dixième extraction est de 100 %.

* Elimination de l'eau

La phase supérieure obtenue après la dixième extraction (environ 25 kg) est soumise à une distillation dans les conditions suivantes :
- pression : 660 - 800 Pa,
- température : 50° C.

On élimine ainsi 65 % de l'eau.

On charge ensuite 25 kg de toluène et on poursuit la distillation sous une pression de 1 300 Pa.

* Elimination des impuretés salines

A la suspension toluénique précédente (environ 33 kg), on ajoute à chaud (50° C) sous agitation 37,5 kg d'acétate d'éthyle. Après refrodissement, on filtre les sels insolubles sur un lit de Clarcel (2 kg) sous une pression de 2,5 bars d'azote.

Le gâteau est lavé avec deux fois 2,5 kg d'acétate d'éthyle.

Le filtrat (70 kg) contient 2,8 kg de triflinate.

* Contre extraction à l'eau

Le filtrat précédent est extrait par 7,5 kg d'eau.

La phase organique contenant le toluène et l'acétate d'éthyle est distillée et chacun des composants est recyclé.

La phase organique (environ 11 kg) contenant le triflinate est distillée sous 660 Pa à une température de 60° C. On obtient une solution aqueuse de 5,5 kg contenant 2,8 kg de triflinate.

2-1 Synthèse du trifluorométhanesulfonate de sodium (triflate de sodium)

Dans un réacteur de 20 litres, balayé à l'azote, contenant la solution aqueuse (5,5 kg) de triflinate (18 moles) et chauffé à 50° C environ, on ajoute lentement 2,24 kg d'eau oxygénée à 30 % (p/p).

En fin de coulée, on augmente la température à 60-65° C et on maintient cette température 1 heure 30 minutes.

Le rendement d'oxydation est quantitatif. On obtient 7,7 kg d'une solution aqueuse contenant 3,1 kg de triflate.

2-2 Traitement de la solution de triflate

Dans un réacteur de 20 litres, on introduit la masse réactionnelle précédente. On effectue une distillation sous une pression de 800 Pa en maintenant la température entre 45 et 50° C. On charge ensuite 5,5 kg de toluène de façon à poursuivre la distillation de l'eau sous forme azéotropique à une température de 35-40° C sous la même pression que précédemment. La masse réactionnelle est ensuite filtrée et le gâteau lavé avec 1,5 kg de toluène, puis séché en étuve (60° C). On obtient 3,15 kg de solide sec contenant environ 96 % de triflate.

3 Synthèse de l'acide trifluorométhane sulfonique (acide triflique)

Dans un réacteur de 20 litres en verre, équipé d'une colonne à distiller, on charge sous azote 4,3 kg d'oléum contenant :
- 3,46 kg de $H_2SO_4$,
- 0,84 kg de $SO_3$.

On coule ensuite, sous agitation, le triflate de sodium sec tout en maintenant la température homogène. En fin d'addition, on porte le milieu à une température d'environ 90° C et on distille l'acide trifluorométhanesulfonique sous un vide de 130 Pa. En fin de distillation, la température est augmentée aux environ de 140° C.

On obtient une masse d'acide triflique de 2,56 kg présentant une pureté de 100 % environ (rendement de la synthèse 3 = 97 %).

EXEMPLE 2

1-1 Synthèse du trifluorométhanesulfinate de sodium

1-2 Traitement du brut réactionnel selon le deuxième mode de mise en oeuvre

On introduit 10,1 kg de brut réactionnel de l'étape 1-1 dans une turbosphère MORITZ TS10 (surface 0,2 $m^2$ - volume 10 litres).

On élimine les solvants DMF/eau dans les conditions suivantes :
- pression : 400 Pa
- température : 60-70° C
- vitesse d'agitation : 200 tours/minute
Sur la poudre obtenue, on ajoute 4,5 kg d'acétate d'éthyle.

6

On chauffe à 50° C sous agitation (100 tours/minute) pendant 20 minutes environ. On laisse refroidir à température ambiante, on filtre les sels insolubles sur lit de Clarcel (0,5 kg) sous une pression de 2,5 bars d'azote.

Le gâteau est lavé avec deux fois 0,75 kg d'acétate d'éthyle.

Le filtrat et les solutions de lavage contenant le triflinate concentrée de façon à obtenir 2 kg de solution aqueuse contenant environ 1 kg de triflinate.

On renouvelle cette étape de traitement trois fois de façon à obtenir 6 kg de solution aqueuse contenant 2,9 kg de triflinate.

2-1 Synthèse du trifluorométhanesulfonate de sodium

On oxyde de la même manière qu'à l'exemple 1, étape 2-1, les 6 kg de solution aqueuse avec 2,32 kg d'eau oxygénée à 30 % (p/p).

Le rendement de l'oxydation est quantitatif. On obtient 8,32 kg de solution aqueuse contenant 3,2 kg de triflate.

2-2 Traitement de la solution de triflate

A la solution précédente, on ajoute 260 g de sulfate ferreux de façon à éliminer l'eau oxygénée excédentaire.

La solution aqueuse résultante (8,6 kg) est introduite dans la même turbosphère qu'à l'étape 1-2 dans les conditions suivantes :
- pression : 400 Pa
- température : 50-60° C
- vitesse de rotation : 200 tours/minute

On obtient 3,6 kg de masse sèche que l'on engage dans l'étape 3 d'acidification.

EXEMPLE 3

Rôle du solvant dans l'extraction solide-liquide

Mode opératoire :

Charger : 25 g environ de l'extrait sec obtenu par filtration de la suspension de toluène dans l'exemple 1-2 avant l'étape d'élimination des impuretés salines, 100 g de solvant.

Agiter 20 à 30 minutes environ.

Filtrer la suspension.

Doser les espèces salines dans la solution filtrée et dans le gâteau préalablement séché.

| Résultats : | | | | |
|---|---|---|---|---|
| | % EXTRAIT DANS LE SOLVANT | | | |
| COMPOSES | MIBK | BUTANOL-1 | ACOEt | ACOEt/O CH$_3$ |
| NaBr | 2,3 % | 14,3 % | ≪ 1 % | < limite détection |
| NaHSO$_3$ | 7,31 % | 5,96 % | 7,99 % | 7, 14 % |
| Na$_2$S$_2$O$_3$ | Non dosable | 8,33 % | 0 % | Non dosable |
| Na$_2$HPO$_4$ | | | | |
| NaH$_2$PO$_4$ | Non dosable | Non dosable | 1,35 % | Non dosable |
| CF$_3$SO$_2$Na | 89,8 % | 96,54 % | > 82,4 % | 87,5 % |

Les résultats obtenus sont issus de dosages en chromatoionique.

Conclusion :

- La MIBK et le Butanol -1 purs semblent être de bons solvants du triflinate. Cependant, le taux en bromure de sodium est encore important. Il est à signaler également que la filtration avec la MIBK est <u>très</u> difficile.
- La présence de toluène dans l'acétate d'éthyle améliore la sélectivité.


3.2 <u>Filtration des impuretés salines</u>

Cette exemple vise à déterminer les conditions d'obtention du triflinate à partir de la suspension toluénique réelle (toluène + sels) :
- Rôle du rapport acétate d'éthyle / toluène compatible avec la filtration des impuretés salines et la qualité du triflinate obtenu dans les jus mères.


Mode opératoire :

A partir de la solution toluénique spécifiée en 3.1.
- Ajout de l'acétate d'éthyle (à chaud : 50°C) dans la suspension saline ainsi obtenue,
- Laisser refroidir à environ 20°C tout en agitant 20 à 30 minutes,
- Filtrer sur filtre N° 2 (500 ml - surface filtrante 78,5 cm²) sous légère dépression (100 à 200 mmHg) pour éviter de flasher l'acétate d'éthyle et colmater ainsi le filtre,
- Laver le gâteau avec 2 fois 50 ml d'acétate d'éthyle,
- Doser les sels solubilisés dans les jus mères de filtration.

| Résultats : | | | |
|---|---|---|---|
| N° DE L'ESSAI | JP 143 | JP 142 | JP 130 |
| JP 117 | 500 | 500 | 500 |
| CHARGES TOLUENE | 500 | 200 | 500 |
| (9) puis ACOet | 200 | 750 | 750 |
| Vitesse Agitation | 600 t/mn | 600 t/mn | 600 t/mn |
| * Nature de la filtration | très mauvaise | moyenne | bonne |
| Masse de gâteau sec | 73,65 g | 69,4 g | 73,9 g |
| Aspect du solide | très fin | fin | fin |

* Cet aspect, bien que très qualitatif permet d'appréhender les difficultés majeures de la filtration.


Par exemple :
- Un bonne filtration dans les conditions définies précédemment dure en moyenne 30 à 45 minutes,
- Une mauvaise filtration dure plusieurs heures.
Dans l'essai JP 143, on observe un "gel" qui colmate rapidement le filtre.
Le dosage des sels a été effectué sur le meilleur essai de filtration (JP 130), c'est-à-dire : ACOet/toluène = 1,5 (pds).

8

| Bilan : | | | |
|---|---|---|---|
| Charge initiale (en sels dans la solution toluénique) | | Masse de gâteau "sec" | Masse de sels dans les jus de filtration |
| NaBr | | | NaBr ≪ 0,1 g |
| NaHSO₃ | | | |
| Na₂S₂O₃ | | | Autres sels < limite détection |
| NaH₂PO₄ Na₂HPO₄ HCO₂Na DMA | 70,8 g | 73,9 g | HCO₂Na : 0,687 g DMA : 1,35 g |
| CF₃SO₂Na | 55 g | | CF SO Na : 54,72 g |
| TOTAL | 125,8 g | 73,9 g | 56,76 g |

Les dosages ont été effectués en chromato-ionique (le titre en triflinate est confimé en RMN F).
Le solide filtré n'est pas complètement sec, ce qui explique l'écart à 10 %.
Le rendement de récupération du triflinate est très bon :

$$\text{Rdt} = \frac{54,72}{55} = 99,5\ \%$$

Le taux de bromure obtenu est très faible < 0,17 %.

## Revendications

1. Procédé de récupération de l'acide triflinique caractérisé par le fait qu'il comporte l'étape de mise en contact d'un mélange de solides contenant au moins un sel de l'acide halohydrique et un sel de l'acide triflinique avec une phase organique contenant :
    - a) un solvant polaire non miscible à l'eau en toute proportion choisi parmi le groupe constitué par les alcools, les cétones et les esters dont le point d'ébullition sous pression atmosphérique est inférieur à 200° C, de préférence à 150° C, et leurs mélanges ;
    - b) un solvant aromatique choisi dans le groupe des solvants dont le point d'ébullition est inférieur à 200° C, de préférence à 150° C et leurs mélanges.

2 . Procédé selon la revendication 1 caractérisé par le fait qu'elle comporte en outre une extraction du diméthylformamide à contre courant avec du dichlorométhane, puis une élimination azéotropique de l'eau avec du toluène.

3. Procédé industriel d'extraction et de purification de trifluorométhanesulfinate obtenu selon le brevet EP 237446, caractérisé en ce que l'on élimine conconmmitamment l'eau et le diméthylformamide par évaporation dans une turbosphère, suivie d'une extraction de la phase résultante par l'acétate d'éthyle et évaporation de cette solution.

4. Procédé selon la revendication 3, caractérisé en ce que le chauffage de la turbosphère est compris entre 50 et è0° C.

5. Procédé de préparation du trifluorométhanesulfonate, caractérisé en ce que le trifluorométhanesulfinate, obtenu selon les revendications 2 ou 3, est oxydé à l'aide d'eau oxygénée puis l'eau éliminé par une des techniques suivantes : distillation azéotropique à l'aide de toluène ou séchage à l'aide d'une turbosphère ou par atomisation.

6. Procédé de préparation de l'acide trifluorométhanesulfonique, caractérisé en ce que l'on met en contact le trifluorométhanesulfonate, obtenu selon la revendication 4, avec un oléum sulfurique contenant au moins 50 % d'excès molaire de trioxyde de soufre par rapport à la quantité d'eau contenue dans le trifluorométhanesulfonate et contenant environ 2 moles d'acide sulfurique par mole de trifluorométhanesulfonate.

FIGURE 1

Office européen
des brevets

**RAPPORT PARTIEL
DE RECHERCHE EUROPEENNE**
qui selon la règle 45 de la Convention sur le brevet
européen est considéré, aux fins de la procédure ultérieure,
comme le rapport de recherche européenne

Numéro de la demande

EP 90 40 1161

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | EP-A-0 237 446 (RHONE-POULENC CHIMIE) <br><br> * Page 3, paragraphe 5; exemples * <br><br>------ | 1-6 | C 07 C 313/04 <br> C 07 C 303/32 <br> C 07 C 309/06 |

**DOMAINES TECHNIQUES RECHERCHES (Int. Cl.4)**

C 07 C 313/00
C 07 C 303/00
C 07 C 309/00

### RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:
Revendications ayant fait l'objet de recherches incomplètes: 1-6
Revendications n'ayant pas fait l'objet de recherches:
Raison pour la limitation de la recherche:

Les revendications de la demande contiennent un nombre d'imprecisions et d'erreurs, ce qui rend une recherche basée sur les revendications impossible. La recherche a donc été basée sur les exemples de la demande et également sur les revendications du premier dépôt, la demande francaise 8905809. La rédaction des revendications peut avoir comme conséquence que la demande ne satisfait pas aux art. 82(unité) et 84(clarté) de la CBE. En plus, la rev. 3 n'est pas compréhensible sans un document additionnel.

| Lieu de la recherche | Date d'achèvement de la recherche | Examinateur |
|---|---|---|
| La Haye | 10-07-1990 | VAN GEYT |